Europäisches Patentamt

European Patent Office

Office européen des brevets

(19)

(11) Numéro de publication : **0 511 912 A1**

## (12) DEMANDE DE BREVET EUROPEEN

(21) Numéro de dépôt : **92401206.5**

(22) Date de dépôt : **28.04.92**

(51) Int. Cl.⁵ : **C12N 15/81,** C12N 1/19, C12P 21/02

(30) Priorité : **30.04.91 FR 9105294**

(43) Date de publication de la demande :
**04.11.92 Bulletin 92/45**

(84) Etats contractants désignés :
**PT**

(71) Demandeur : **RHONE-POULENC RORER SA**
**20, avenue Raymond Aron**
**F-92160 Antony (FR)**

(72) Inventeur : **Fleer, Reinhard**
**1 Allée Port Royal, Résidence de l'Abbaye**
**F-91190 Gif Sur Yvette (FR)**
Inventeur : **Fournier, Alain**
**28 Avenue Roger Salengro**
**F-92000 Chatenay Malabry (FR)**
Inventeur : **Mayaux, Jean-François**
**21 ter Boulevard de la République**
**F-92260 Fontenay aux Roses (FR)**
Inventeur : **Yeh, Patrice**
**11bis, rue Lacépède**
**F-75005 Paris (FR)**

(74) Mandataire : **Savina, Jacques et al**
**Rhône-Poulenc Rorer S.A. Direction Brevets**
**(t144) 20 avenue Raymond Aron**
**F-92165 Antony Cédex (FR)**

(54) **Promoteur de levure et son utilisation.**

(57)   L'invention concerne des séquences d'ADN comprenant tout ou partie du promoteur du gène PGK de K.lactis, ou d'un dérivé de celui-ci, et possèdant une activité de promoteur transcriptionnel. Elle concerne également l'utilisation de ces séquences pour l'expression de gènes recombinés.

FIGURE 3

La présente invention concerne le domaine de la biologie moléculaire. Plus particulièrement, elle concerne une nouvelle séquence d'ADN présentant une activité de promoteur transcriptionnel, des vecteurs d'expression contenant cette séquence, et son utilisation pour la production de protéines, et par exemple de protéines hétérologues. L'invention concerne aussi les cellules recombinées contenant cette séquence d'ADN.

Les progrès accomplis dans le domaine de la biologie moléculaire ont permis de modifier des microorganismes pour leur faire produire des protéines hétérologues. En particulier, de nombreuses études génétiques ont porté sur la bactérie E. coli. Toutefois, l'application industrielle de ces nouveaux modes de production est encore limitée, en particulier par les problèmes d'efficacité d'expression des gènes dans ces microorganismes recombinés. Aussi, dans le but d'augmenter les performances de ces systèmes de production, des recherches ont été effectuées afin d'isoler des promoteurs forts, permettant d'obtenir des niveaux élevés d'expression de protéines hétérologues. Chez E.coli, on peut citer en particulier les promoteurs des opérons tryptophane et lactose.

Plus récemment, chez la levure S. cerevisiae, des études ont porté sur des promoteurs dérivés de gènes impliqués dans la glycolyse. On peut citer notamment les travaux sur le promoteur du gène de la 3-phosphoglycerate kinase PGK (Dobson et al., Nucleic Acid Res. 10, 1982, 2625 ; Hitzeman et al., Nucleic Acid Research 1982, 7791), sur celui du gène de la glyceraldéhyde-3-phosphate déshydrogénase GAPDH (Holland et al., J.Biol.Chem. 254, 1979, 9839 ; Musti et al., Gene 25, 1983, 133), sur celui du gène de l'alcool déshydrogénase 1 ADH1 (Bennentzen et al., J.Biol.Chem. 257, 1982, 3018 ; Denis et al., J.Biol.Chem. 25, 1983, 1165), ou sur celui du gène de l'enolase 1 ENO1 (Uemura et al., Gene 45, 1986, 65).

Récemment, des outils génétiques ont été développés afin de se servir de la levure Kluyveromyces comme cellule hôte pour la production de protéines recombinantes. La mise en évidence d'un plasmide de type 2-micron originaire de K-drosophilarum (plasmide pKD1 - EP 241 435) a permis d'établir un système hôte/vecteur très efficace pour la production de protéines recombinantes (EP 361 991). Cependant, les promoteurs utilisés dans ce système n'ont jamais été optimisés. En particulier, il s'agit essentiellement de promoteurs hétérologues, c'est-à-dire provenant d'autres microorganismes, tel que notamment S.cerevisiae. Cette situation peut engendrer différents inconvénients, et notamment limiter l'activité du promoteur à cause de l'absence de certains éléments de la machinerie transcriptionnelle (par exemple de transactivateurs), présenter une certainee toxicité pour la cellule hôte due à une absence de régulation, ou affecter la stabilité du vecteur.

Dans ces conditions, le manque de promoteurs homologues forts chez Kluyveromyces constitue un facteur limitant dans l'exploitation industrielle de ce système d'expression.

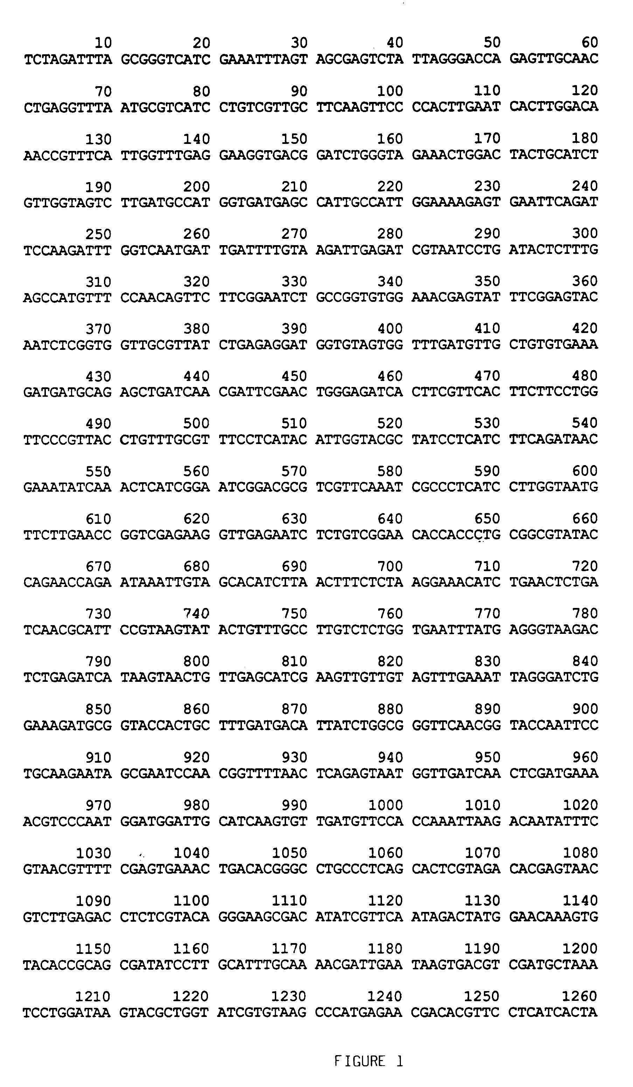

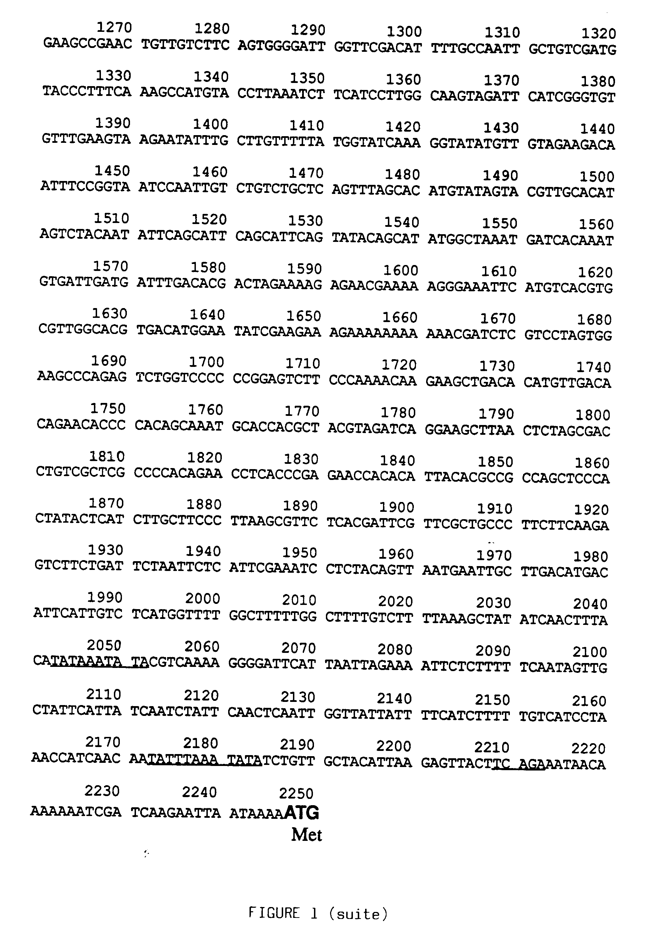

La Demanderesse a maintenant identifié, cloné et séquencé une région du génome de Kluyveromyces lactis présentant une activité de promoteur transcriptionnel (voir figure 1). Plus précisément, cette région correspond au promoteur du gène PGK de K.lactis. Cette région, ou des dérivés ou fragments de celle-ci, peut être utilisée de manière très performante pour la production de protéines recombinantes chez les levures du genre Kluyveromyces. Il est entendu que cette séquence peut également être utilisée dans d'autres organismes hôtes.

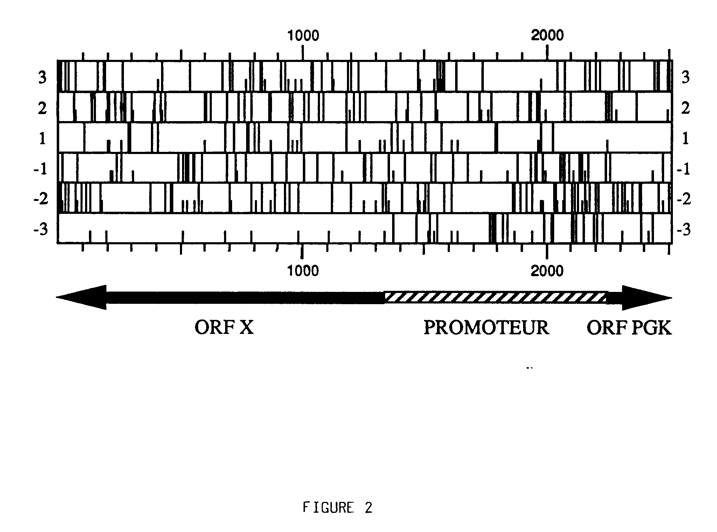

Par ailleurs, l'analyse de la région du génome de Kluyveromyces obtenue a permis de mettre en évidence 2 phases de lecture dans les 2 orientations opposées (voir figure 2). Cette observation indique que le brin complémentaire de la région présentée sur la figure 1 possède également une activité promotrice agissant dans l'autre orientation.

Un objet de la présente invention réside donc dans une séquence d'ADN comprenant tout ou partie de la séquence présentée à la figure 1 ou de son brin complémentaire, ou d'un dérivé de celles-ci, et possédant une activité de promoteur.

Au sens de la présente invention, on entend par dérivé, toute séquence obtenue à partir de la séquence donnée dans la figure 1 par modifications structurales (mutations, délétions, substitutions, additions, fragmentations ... ) conservant une activité de promoteur. En particulier, les mutations peuvent porter sur un ou plusieurs nucléotides, et les additions et/ou substitutions peuvent porter sur des éléments de régulation, ou des régions activatrices telles que les "UAS".

Lorsqu'un dérivé est réalisé, son activité de promoteur transcriptionnel peut être mise en évidence de plusieurs façons, et en particulier en plaçant sous le contrôle de la séquence étudiée, un gène de résistance ou un marqueur de complémentation. Toute autre technique connue de l'homme de l'art peut bien évidemment être utilisée à cet effet.

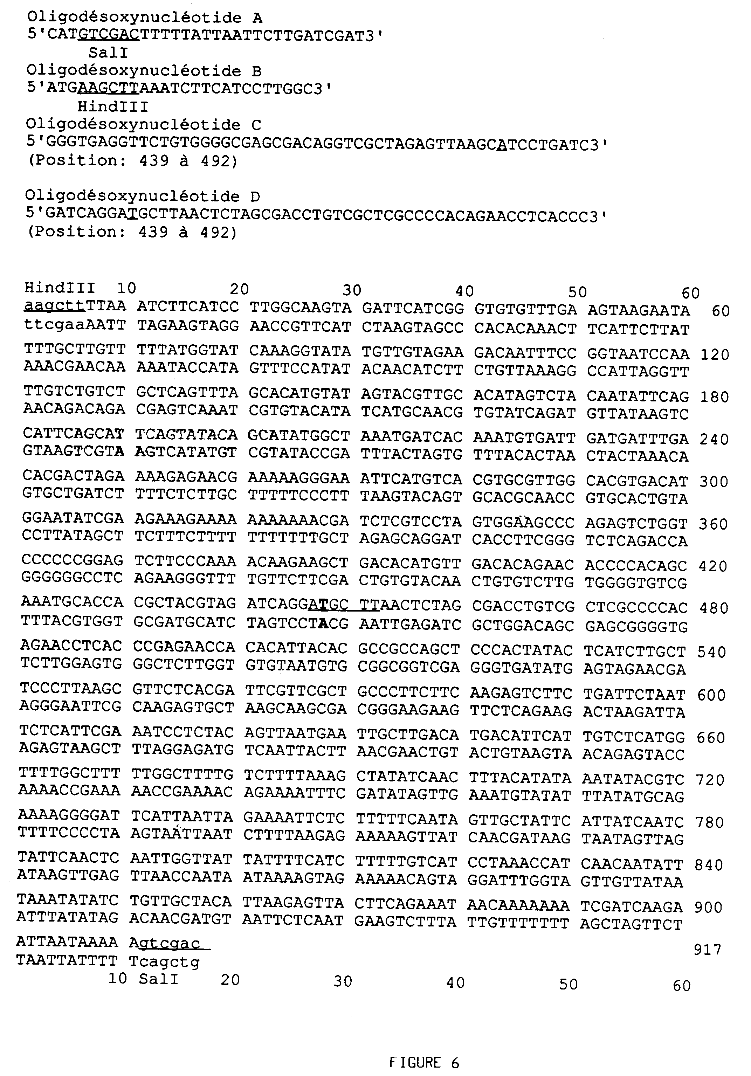

Un objet plus particulier de l'invention concerne une séquence d'ADN correspondant à la région comprise entre les 2 phases ouvertes ORF PGK et ORF X, telle que présentée sur la figure 6.

Un autre objet de l'invention concerne un ADN recombinant comprenant une séquence d'ADN telle que définie ci-dessus.

Cet ADN recombinant peut contenir par exemple la séquence promotrice présentée à la figure 1 ou un dérivé de celle-ci, dans laquelle est inséré un site de restriction, facilitant l'utilisation de cette séquence comme promoteur, "portable".

Préférentiellement, cet ADN recombinant contient en outre un ou plusieurs gènes de structure.

Encore plus préférentiellement, l'ADN recombinant contient également des signaux permettant la sécrétion du produit d'expression du ou desdits gènes de structure.

Dans un mode de réalisation particulier de l'invention, l'ADN recombinant fait partie d'un plasmide d'expression, qui peut être à réplication autonome ou intégratif.

En particulier, des vecteurs à réplication autonome peuvent être obtenus en utilisant des séquences à réplication autonomes (ARS) chez l'hôte choisi. Notamment, chez la levure, il peut s'agir d'origines de réplication dérivées de plasmides connus (pKD1, 2μ, etc).

Les vecteurs intégratifs peuvent être obtenus notamment en utilisant des séquences homologues à certaines régions du génome de l'hôte, permettant, par recombinaison homologue, l'intégration du vecteur.

La séquence présentée sur la figure 1 a été obtenue par criblage d'une banque d'ADN génomique total de Kluyveromyces lactis au moyen d'une sonde hétérologue provenant du gène PGK de S. cerevisiae. La Demanderesse a en effet montré qu'il est possible de cloner une région promotrice chez Kluyveromyces, par hybridation à partir de sondes hétérologues correspondant à un gène de S. cerevisiae. Les détails du clonage de la séquence sont donnés dans les exemples. La région intergénique peut ensuite être isolée à partir de cette séquence, notamment par insertion de sites de restriction en utilisant la technique d'amplification par PCR comme indiqué dans les exemples.

Un autre objet de l'invention concerne les cellules recombinées contenant une séquence d'ADN telle que définie ci-avant.

Avantageusement, les cellules sont choisies parmi les levures, et encore plus préférentiellement, parmi les levures du genre Kluyveromyces. Il est entendu cependant que l'invention couvre toute les cellules recombinées dans lesquelles les régions promotrices de l'invention sont actives.

Ces cellules peuvent être obtenues par toute méthode permettant d'introduire un ADN étranger dans une cellule. Il peut s'agir notamment de transformation, électroporation, ou toute autre technique connue de l'homme de l'art.

Un autre objet de l'invention concerne l'utilisation d'une séquence telle que précédemment définie pour l'expression de gènes recombinés.

Comme l'illustrent les exemples, les séquences d'ADN selon l'invention permettent en effet une production à des niveaux élevés de protéines recombinantes.

Par ailleurs, l'activité promotrice bidirectionnelle des séquences de l'invention permet une utilisation particulièrement avantageuse. Notamment, il est possible d'utiliser ces séquences dans les 2 orientations possibles, pour l'expression simultanée de plusieurs gènes de structure.

Avantageusement, l'invention concerne l'utilisation d'une séquence telle que précédemment définie pour l'expression simultanée, dans les 2 orientations opposées, de gènes recombinés.

Avantageusement, les séquences de l'invention peuvent être utilisées pour l'expression de gènes codant pour des protéines d'intérêt pharmaceutique ou agroalimentaire. A titre d'exemple, on peut citer les enzymes (tels que notamment la superoxide dismutase, la catalase, les amylases, les lipases, les amidases, la chymosine etc.), les dérivés sanguins (tels que la sérum-albumine, l'alpha- ou la béta-globine, le facteur VIII, le facteur IX, le facteur van Willebrand, la fibronectine, l'alpha-1 antitrypsine etc.), l'insuline et ses variants, les lymphokines (telles que les interleukines, les interférons, les facteurs de stimulation des colonies (G-CSF, GM-CSF, M-CSF...), le TNF, le TRF etc.), les facteurs de croissance (tels que l'hormone de croissance, l'érythropoïétine, le FGF, l'EGF, le PDGF, le TGF etc.), les apolipoprotéines, des polypeptides antigéniques pour la réalisation de vaccins (hépatite, cytomégalovirus, Eppstein-Barr, herpes etc.), ou encore des fusions de polypeptides telles que notamment des fusions comportant une partie active fusionnée à une partie stabilisatrice (par exemple des fusions entre l'albumine ou des fragments d'albumine et le récepteur ou une partie d'un récepteur de virus (CD4, etc)).

L'invention sera plus complètement décrite à l'aide des exemples qui suivent, qui doivent être considérés comme illustratifs et non limitatifs.

LEGENDE DES FIGURES

Figure 1 : Séquence nucléotidique de la région de 2,2 kb du fragment chromosomique situé en amont du codon d'initiation de la traduction du gène PGK de K.lactis possédant l'activité promotrice.

Figure 2 : Analyse des phases ouvertes de lecture. Les demi-traits verticaux représentent des codons d'initiation de la traduction. Les traits verticaux entiers représentent des codons stop. Les régions claires mettent en évidence les phase ouvertes de lecture (ORF X et ORF PGK).

Figure 3: Carte de restriction du plasmide pYG610. La région noire correspond à la région isolée du génome de K.lactis.

Figure 4: Stratégie de séquençage du fragment Xbal de 2,5 kb.

Figure 5 : Séquence et localisation des oligodéoxynucléotides utilisés dans la réaction de PCR, pour l'insertion d'un site HindIII en -6 de l'ATG de la séquence de la figure 1. Les oligodéoxynucléotides sont représentés en italique. L'ATG correspond au codon d'initiation de la traduction du gène PGK.

Figure 6 : Séquence nucléotidique de la région intergénique du fragment 2,2 kb. 6(a) : oligodéoxynucléotides utilisés dans la réaction de PCR. 6(b) : Fragment Sal(I)-HindIII correspondant aux nucléotides 1343 à 2246 sur la séquence de la figure 1.

Figure 7: Stratégie de construction du plasmide pYG45.

Figure 8: Stratégie de construction de cassettes d'expression de la sérum-albumine humaine.

Figure 9: Stratégie de construction du plasmide pYG65.

Figure 10: Stratégie de construction du plasmide pYG70.

Figure 11: Stratégie de construction du plasmide pYG72.

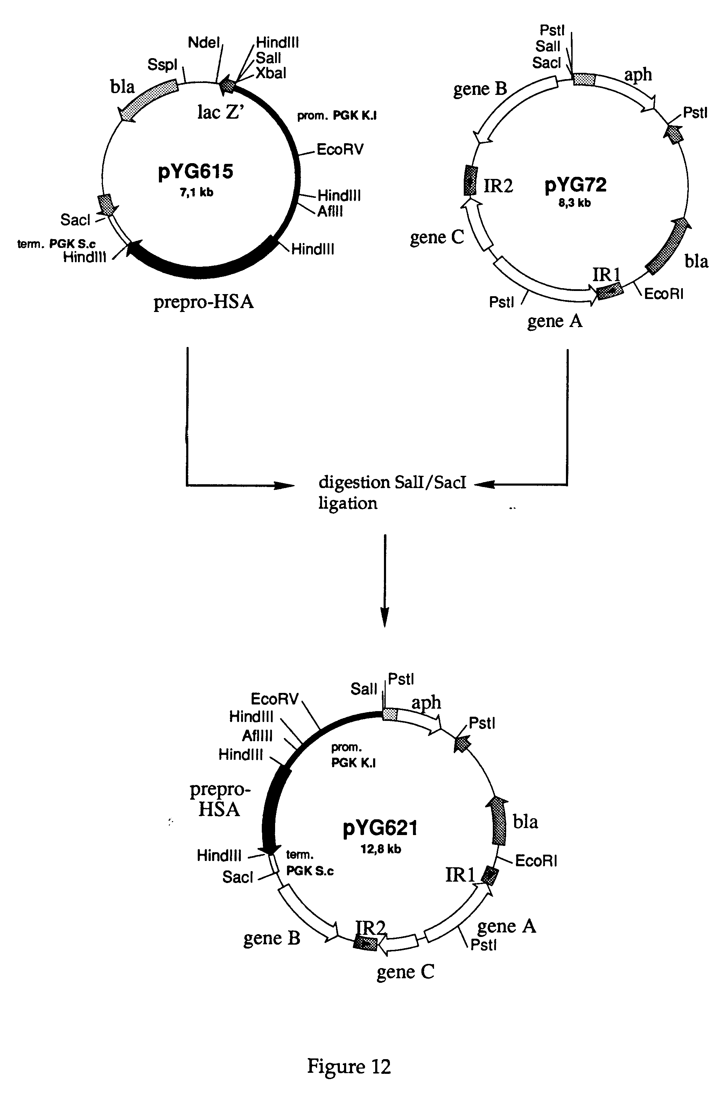

Figure 12: Stratégie de construction du vecteur pYG621.

Figure 13 : Mise en évidence par "Northern blot" de l'expression du gène de l'albumine humaine sous la dépendance du promoteur PGK de K.lactis. Les échantillons correspondent à 10µg d'ARN total. 18S et 28S sont les positions des ARN ribosomiques 18S et 28S. ALB = fragments reconnus par la sonde correspondant au gène de l'albumine; URA = fragments reconnus par la sonde correspondant au gène URA A de K.lactis servant de témoin de dépôt.

Figure 14 : Mise en évidence de la production d'albumine dans les souches transformées par le vecteur d'expression pYG621 contenant le promoteur PGK de K-lactis. Les échantillons correspondent à 30 µl de sumageant de culture ; les bandes au niveau du marqueur 66 kd correspondent à l'albumine. M = marqueurs de masse moléculaire : anhydrase carbonique bovine (31Kd), ovalbumine (45Kd), BSA (66Kd), phosphorylase b de lapin (92kd).

EXEMPLES

1/ Isolement de la région promotrice du gene PGK de K lactis.

La séquence présentée sur la figure 1 a été obtenue par criblage d'une banque d'ADN génomique total de Kluyveromyces lactis CBS2359 au moyen d'une sonde hétérologue provenant du gène PGK de S. cerevisiae (Dobson et al., Nucleic Acid Res. 1982, 10, 2625). Plus précisément, la sonde utilisée correspond au fragment N-terminal PvuI-EcoRI de 1,3 kb du gène PGK de S. cerevisiae.

En "Southern blot" (Southern et al., J.Biol.Chem., 1975, 98, 503), la sonde utilisée s'hybride avec deux fragments différents lorsque l'ADN génomique est digéré par XBaI. L'un d'eux, de 2,5 kb environ, a été isolé par criblage d'une banque génomique restreinte de K. lactis CBS2359, constituée de fragments d'ADN coupés par XbaI, d'une taille comprise entre 2 et 3 kb, introduits dans le plasmide pUC18 au site XbaI. Une banque de 500 clones a ainsi été constituée, puis criblée avec la sonde hétérologue décrite ci-dessus.

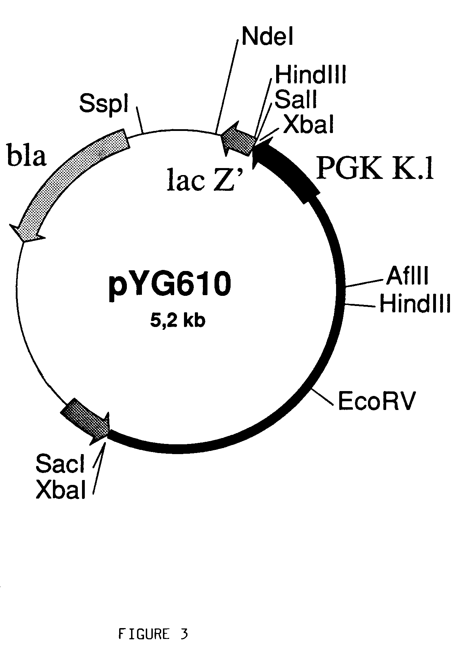

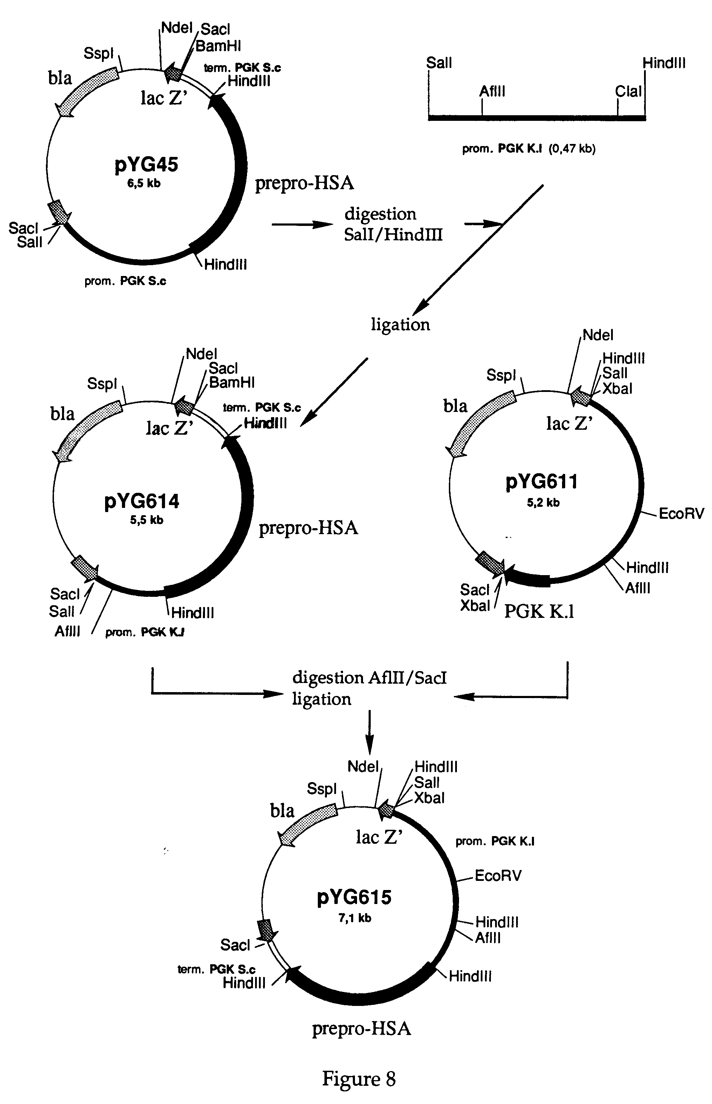

Par hybridation sur colonies, un clone a pu être identifié et son ADN plasmidique a été préparé. Ce plasmide (pYG610) contient un fragment d'ADN génomique de 2,5 kb, dont la carte de restriction est présentée à la figure 3. Le plasmide pYG611 contient le même insert dans la direction opposée (voir figure 8).

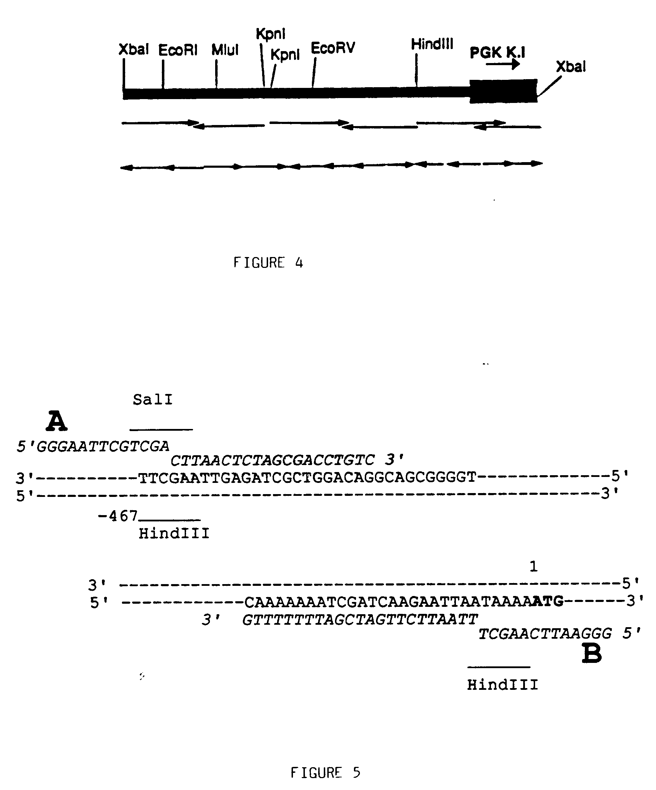

Dans une seconde étape, le fragment de 2,5 kb ainsi isolé a été séquencé, en utilisant la méthode de Sanger (Sanger et al., Proc.Nat.Acad.Sci 74, 1977, 5463). Pour cela, le fragment issu de pYG611 a d'abord été sous-cloné dans les bactériophages M13tg130 et M13_tg131. La stratégie de séquençage du fragment est schématisée sur la figure 4.

L'analyse de la séquence obtenue montre que le fragment isolé contient une partie codant pour la région N-terminale de la protéine Pgk de Kluyveromyces lactis (0,3 kb), et 2,2 kb correspondant à la région promotrice située en amont du site d'initiation de la traduction. Elle montre de plus que, dans l'orientation opposée par rapport au gène PGK, se situe une seconde phase de lecture située à environ 0,9 kb en amont de l'ATG du gène PGK (figure 2).

La comparaison de cette séquence avec celle du promoteur du gène PGK de S. cerevisiae fait apparaitre l'absence d'homologie particulière, notamment avec son élément de régulation. Cette séquence correspond donc à une région promotrice entièrement originale, très distincte de celles déjà décrites, sur le plan de sa structure, et par conséquent sur le plan de sa régulation.

2/ Construction de vecteurs d'expression pour la production de proteines hétérologues :

Cet exemple illustre l'utilisation des capacités promotrices de la séquence de 2,2 kb de la séquence de la figure 1 et de séquences dérivées.

a) Insertion d'un site de restriction en -6 de l'ATG.

L'insertion de ce site permet ensuite d'introduire en aval du promoteur obtenu tout gène que l'on désire exprimer. Pour des raisons de compatibilité avec des vecteurs d'expression existant (EP 361 991), des promoteurs "portables" ont été construits sous forme de fragments SalI-HindIII.

Un site HindIII a été introduit en position -6 par rapport au site d'initiation de la traduction (ATG) du gène PGK en utilisant la technique d'amplification par PCR (Mullis et al., Meth.Enzymol. 155, 1987, 335). Dans ce but, 2 oligodéoxynucléotides ont été utilisés, qui sont présentés sur la figure 5.

L'oligodéoxynucléotide A correspond à la séquence située à 467 pb en amont du codon ATG, au niveau d'un site HindIII, qui sera ainsi remplacé par un site SalI lors de l'amplification. L'oligodéoxynucléotide B correspond à la séquence en amont du site d'initiation, et permet d'introduire un site HindIII en position -6.

Le fragment obtenu par PCR a été inséré entre les sites SalI et HindIII du bactériophage M13tg130 afin de vérifier par séquençage que des mutations ne sont pas apparues lors de l'amplification.

b) Construction de cassettes d'expression de la sérum-albumine humaine : figure 8.

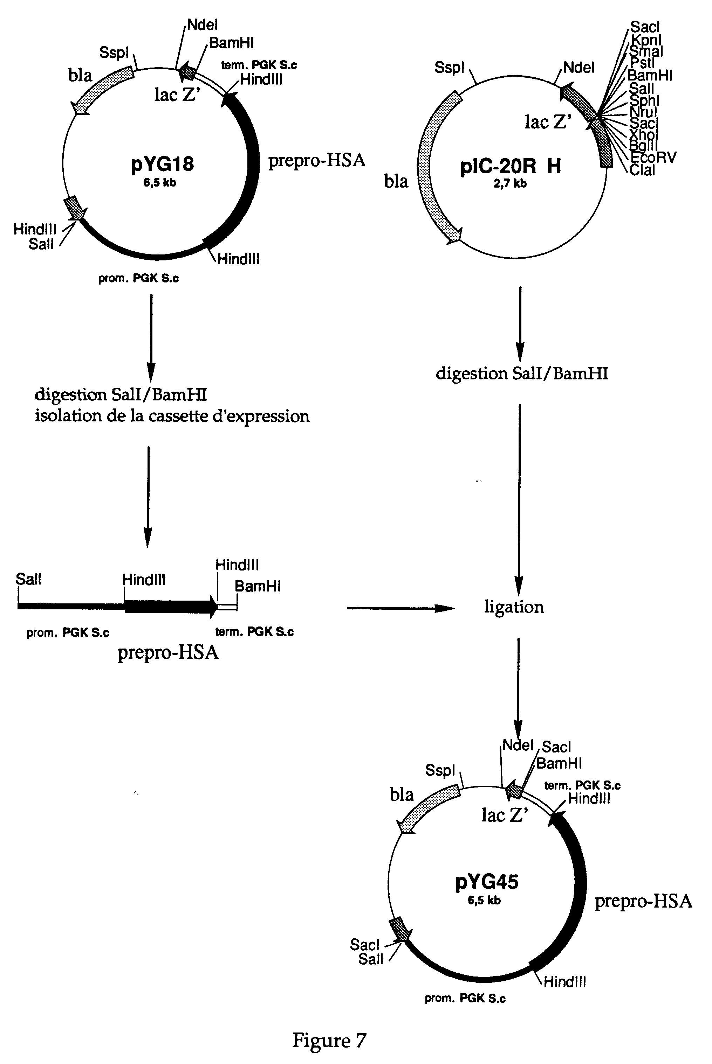

L'ADN recombinant de 474 pb obtenu ci-dessus a été introduit au niveau des sites SalI et HindIII, dans le plasmide pYG45 (figure 7) pour obtenir le vecteur pYG614 (figure 8). Le plasmide pYG45 contient une cassette d'expression constituée du promoteur et du terminateur du gène PGK de S. cerevisiae entre lesquels, au niveau d'un site HindIII, est inséré le gène codant pour la prépro-sérum-albumine humaine (séquence prépro-HSA). pYG45 est dérivé de pYG18 (voir brevet EP 361 991) par sous-clonage du fragment SalI-BamHI contenant la cassette d'expression HSA, dans les sites correspondants du vecteur pIC-20RDH (figure 7). pIC-20RDH est obtenu par digestion du plasmide pIC-20R (March et al., Gene 32, 1984, 481) avec l'enzyme HindIII, remplissage des extrémités avec le fragment Klenow de la polymérase I d'E.coli et recircularisation avec la T4 DNA ligase.

A partir du plasmide pYG614, le fragment SalI-SacI peut être isolé par digestion. Il contient : une région promotrice dérivée de la séquence de la figure 1, le gène de l'albumine et le terminateur du gène PGK de S. cerevisiae. Il constitue une cassette d'expression qui peut être insérée dans un plasmide pour constituer un vecteur d'expression.

Une autre cassette d'expression peut être obtenue à partir du plasmide pYG614, par clonage du fragment AflIII-SacI contenant une partie du promoteur PGK de l'invention, le gène de l'albumine (prépro-HSA) et le terminateur PGK de S. cerevisiae dans le plasmide pYG611 décrit préalablement. Ceci génère le plasmide pYG615. Le fragment SalI-SacI contenant : la région promotrice de la figure 1 entière, le gène codant pour la prépro-sérum-albumine, et le terminateur du gène PGK de S. cerevisiae, peut ensuite être isolé par digestion. Ce fragment constitue une seconde cassette d'expression de l'albumine utilisant la séquence promotrice de l'invention.

c) Construction de vecteurs d'expression de l'albumine.

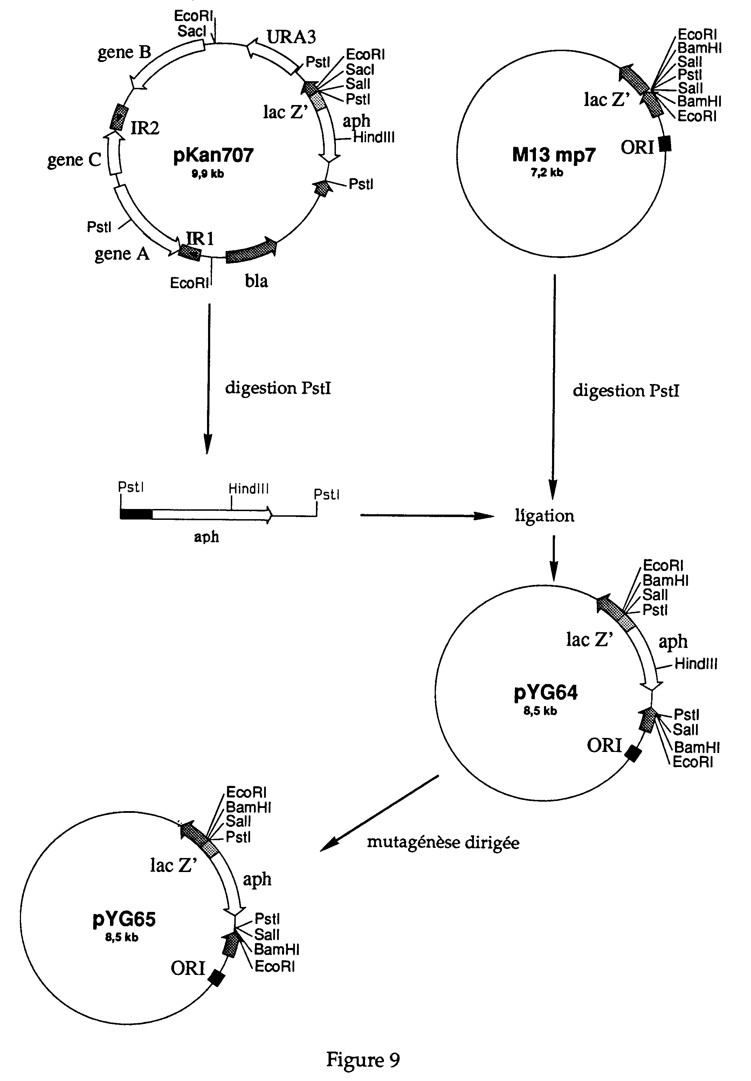

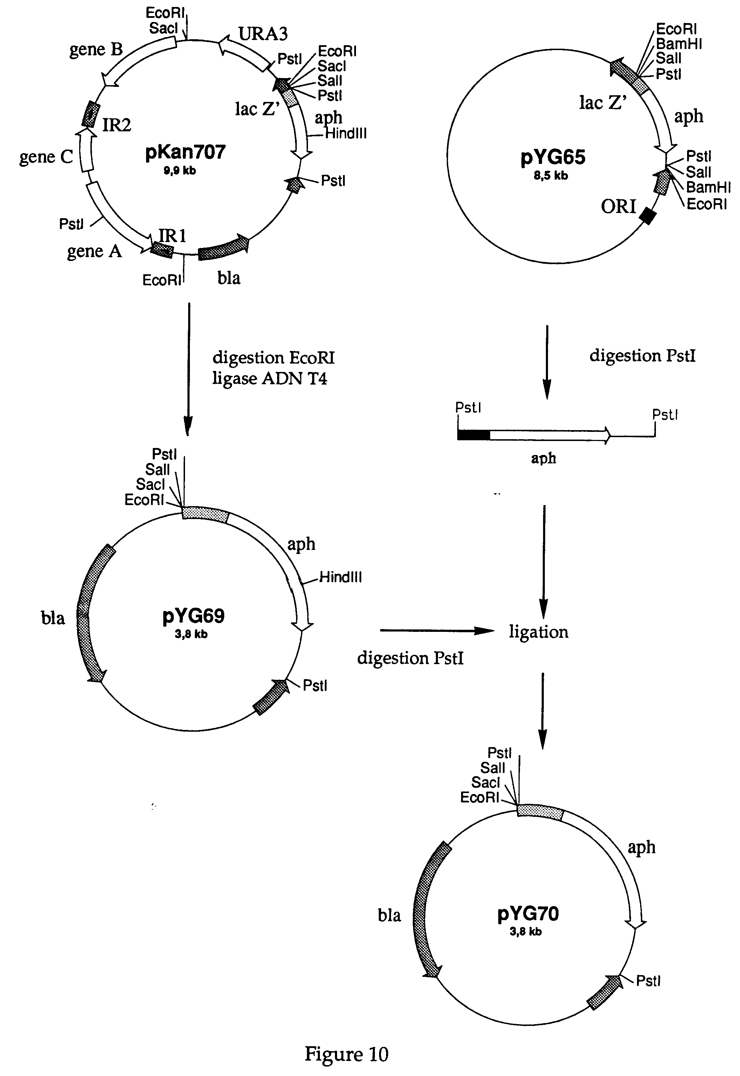

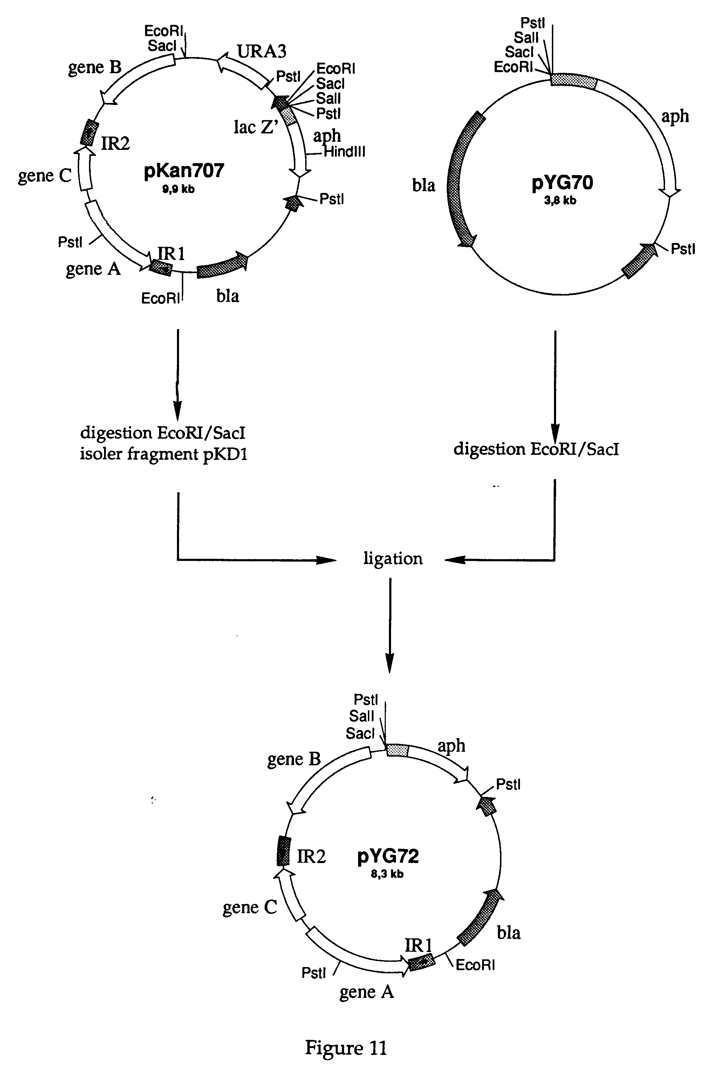

Des vecteurs d'expression de l'albumine peuvent être construits par insertion des cassettes d'expression obtenues ci-dessus dans des plasmides navettes K.lactis/E.coli tels que pYG72 (figure 10). En particulier, un vecteur d'expression a été obtenu (vecteur pYG621) par insertion du fragment SalI-SacI de pYG615 contenant la cassette d'expression de l'albumine dans le vecteur pYG72 (voir figure 10). Ce vecteur correspond au plasmide pKan 707 (voir EP 361 991) dans lequel le fragment SacI contenant le gène URA3 a été éliminé, ainsi que le site unique HindIII présent dans le gène aph pour faciliter les constructions ultérieures. Le gène aph code pour l'aminoglycoside 3'-phosphotransférase (I) (Oka et al., J. Mol-Biol. 147, 1981, 217) et est utilisé comme marqueur de résistance au G418 chez la levure. Le fragment PstI du plasmide pKan707 contenant le gène aph a été sous-cloné dans le bactériophage M13mp7 pour donner le vecteur pYG64 (figure 9). Le site HindIII présent dans ce gène a été détruit par mutagénèse dirigée selon la méthode décrite par Taylor et al. (Nucleic Acid Res. 13, 1985, 8749). Le plasmide résultant a été appelé pYG65 (figure 9). L'oligodéoxynucléotide utilisé pour la mutagénèse avait la séquence 5'-GAA ATG CAT AAG CTC TTG CCA TTC TCA CCG -3' et transformait le triplet CTT codant pour l'acide aminé 185 (Leu) en CTC. Ce changement ne modifie pas la séquence protéique résultante. Pour construire le plasmide pYG72, la partie contenant le réplican bactérien du vecteur pKan707 a été isolée par digestion avec l'enzyme EcoRI et recircularisation avec la T4 DNA ligase pour obtenir pYG69. Le fragment PstI présent dans ce dernier vecteur contenant le gène aph a été remplacé par le fragment équivalent muté provenant de pYG65. Cette construction a été appelée pYG70. La séquence de pKD1 de 4,7 kb comprise entre les sites EcoRI et SacI a été introduite dans ce dernier vecteur pour obtenir pYG72. Le vecteur pYG621 (figure 11) a été obtenu par insertion du fragment SalI-SacI contenant la cassette d'expression de l'albumine provenant de pYG615.

3/ Construction d'une cassette permettant d'utiliser la région promotrice dans les 2 orientations.

Cette construction a été obtenue par introduction d'un site SalI et d'un site HindIII de part et d'autre de la région comprise entre les 2 phases ouvertes de lecture identifiées sur la figure 2: ORF PGK et ORF X, soit au niveau des nucléotides 1343 et 2246 sur la figure 1.

Cette construction a été réalisée par la technique de PCR en utilisant d'une part l'oligodéoxynucléotide A qui introduit un site SalI en position -1 par rapport au site d'initiation de la traduction du gène PGK, et d'autre part l'oligodéoxynucléotide B qui introduit un site HindIII en position -1 par rapport au site d'initiation de la traduction du gène X (voir figure 6(a)). Ensuite, pour éliminer un site HindIII présent dans la région promotrice, 3 réactions de PCR ont été effectuées en utilisant à chaque étape le plasmide pYG610 comme matrice :

- les 2 premières, pour amplifier les régions de part et d'autre du site HindIII en utilisant les oligodéoxynueléotides A et B couplés respectivement aux oligodéoxynucléotides C et D (figure 6). Ces 2 derniers sont complémentaires et permettent d'introduire une mutation ponctuelle au niveau du site HindIII interne ;
- la dernière, en utilisant les 2 produits d'amplification précédents comme amorce, pour générer le fragment final contenant la région promotrice modifiée.

Cette région peut ensuite être introduite dans les vecteurs décrits dans l'exemple 2, et utilisée comme promoteur bidirectionnel.

## 4/ Expression d'albumine

Le vecteur pYG621 a été introduit par transformation dans la souche K.lactis MW98-8C (CBS 579.88), en utilisant la technique éthylène glycol/diméthylsulfoxyde (Durrens et al., 1990, Curr.Genet. 18, 7). Cette souche dérive de la souche sauvage CBS2359 et présente le génotype : Mat_, uraA, LysA, argA, K+, cir. Les levures transformées sont sélectionnées pour le phénotype "G418-resistant" que confère le plasmide pYG621 sur milieu YPD (extrait de levure 10 g/l, peptone 20 g/l, glucose 20 g/l) contenant de la généticine à 0,2 g/l. Des souches transformées par le plasmide pYG72 ne contenant pas de cassette d'expression ont été sélectionnées pour servir de témoin dans les tests de production. Par ailleurs, afin de comparer l'efficacité du promoteur PGK de K.lactis selon l'invention par rapport à celui de S.cerevisiae, des souches transformées par le vecteur pYG19 ont également été sélectionnées. Le vecteur pYG19 est analogue au vecteur pYG621, sauf que le gène de l'albumine est sous le controle du promoteur PGK de S.cerevisiae (EP 361 991).

a) Analyse des ARNm :

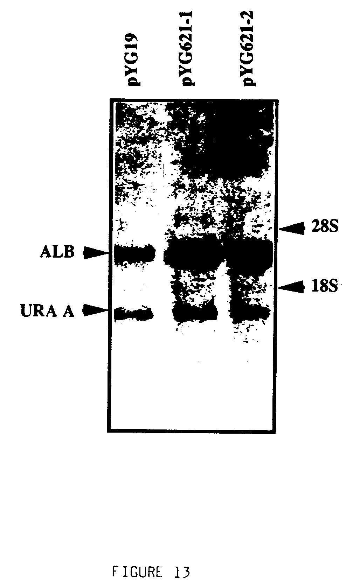

Les cellules sont cultivées à 28°C en milieu sélectif YPD (extrait de levure 10 g/l, peptone 20 g/l, glucose 20 g/l) contenant de la généticine à 0,2 g/l. Les ARN totaux sont extraits (Sherman et al., Methods in Yeast Genetics, Cold Spring Harbor Laboratoty, 1986, 143) et séparés par électrophorèse sur gel d'agarose. Suivant la méthode de "Northern blot" (Maniatis et al., 1982 Molecular cloning, Cold Spring Harbor, Laboratory Press), les ARN sont hybridés à une sonde correspondant au gène de structure de l'albumine (fragment HindIII-HindIII de 1,9 kb) provenant du vecteur pYG18 (figure 7). L'autoradiographie montre clairement une bande de 2,3 kb spécifique de l'albumine (figure 13). Par ailleurs, il apparait clairement que le taux de transcription du gène de l'albumine est bien supérieur dans les souches contenant une région promotrice de l'invention (pYG621), que dans celles contenant le promoteur PGK intact de S. cerevisiae (pYG19).

b) Analyse des protéines :

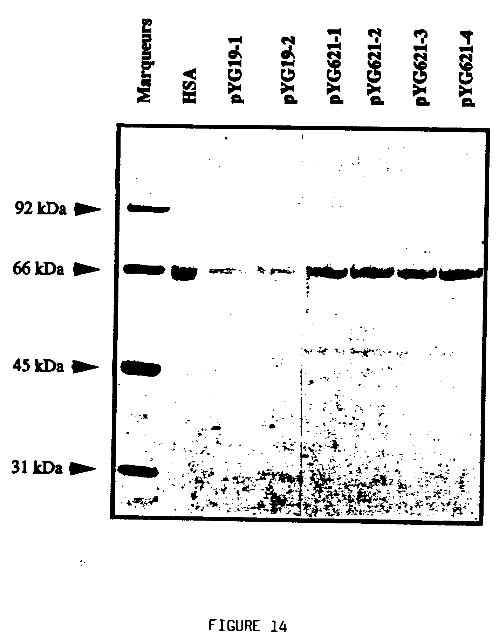

Les cellules sont cultivées en erlenmeyers dans un milieu sélectif YPD (extrait de levure 10 g/l, peptone 20 g/l, glucose 20 g/l) contenant de la généticine à 0,2 g/l à 28°C sous agitation. Après 96 heures de culture, 30 μl de surnageant sont prélevés et mélangés à un volume équivalent de tampon Laemmli 2X (Laemmli, 1970, Nature 227, 680). Après chauffage à 96°C pendant 10 minutes, les protéines de l'échantillon sont ensuite séparées sur gel de polyacrylamide SDS 8,5 %. La production d'albumine est ensuite révélée par coloration du gel au bleu de coomassie, puis est évaluée pour les différents vecteurs utilisés. La figure 14 montre que les 4 clones obtenus séparément par transformation de la souche MW98-8C par le vecteur pYG621 sécrètent beaucoup plus d'albumine que ceux obtenus par transformation avec le vecteur pYG19.

Il est clair que la région promotrice de l'invention permet une excellente production d'albumine par la levure, supérieure à celle obtenue avec le promoteur PGK de S.cerevisiae. Cette région, ou des formes réduites ou dérivées de celle-ci, constituent un outil industriel important pour les systèmes de production microbiologiques, et plus particulièrement eucaryotes.

## Revendications

1. Séquence d'ADN comprenant tout ou partie de la séquence présentée à la figure 1 ou de son brin complémentaire, ou d'un dérivé de celles-ci, et possédant une activité de promoteur transcriptionnel.

2. Séquence d'ADN selon la revendication 1 comprenant tout ou partie de la séquence présentée sur la figure 6(b).

3. ADN recombinant comprenant une séquence d'ADN selon les revendications 1 ou 2.

4. ADN recombinant selon la revendication 3 caractérisé en ce qu'il contient en outre un ou plusieurs gènes de structure.

5. ADN recombinant selon la revendication 4 caractérisé en ce qu'il contient également des signaux permettant la sécretion du produit d'expression du ou desdits gènes de structure.

6. ADN recombinant selon l'une quelconque des revendications 3 à 5 caractérisé en ce qu'il fait partie d'un plasmide d'expression, qui peut être à réplication autonome ou intégratif.

7. Cellule recombinée contenant une séquence d'ADN ou un ADN recombinant selon l'une quelconque des revendications précédentes.

8. Cellule recombinée selon la revendication 7 caractérisée en ce qu'il s'agit d'une levure.

9. Cellule recombinée selon la revendication 8 caractérisée en ce qu'il s'agit d'une levure du genre Kluyveromyces.

10. Utilisation d'une séquence d'ADN selon l'une quelconque des revendications 1 à 6 pour l'expression de gènes recombinés.

11. Utilisation selon la revendication 10 pour l'expression simultanée, dans les 2 orientations opposées, de gènes recombinés.

12. Utilisation selon l'une des revendications 10 ou 11 pour l'expression de gènes codant pour des protéines d'intérêt pharmaceutique ou agroalimentaire.

13. Procédé de préparation d'une protéine recombinante par expression de son gène dans un hôte cellulaire caractérisé en ce que l'expression dudit gène est sous le contrôle d'une séquence selon la revendication 1.

14. Procédé selon la revendication 13 caractérisé en ce que la protéine est la sérum-albumine humaine.

```
         10         20         30         40         50         60
TCTAGATTTA GCGGGTCATC GAAATTTAGT AGCGAGTCTA TTAGGGACCA GAGTTGCAAC

         70         80         90        100        110        120
CTGAGGTTTA ATGCGTCATC CTGTCGTTGC TTCAAGTTCC CCACTTGAAT CACTTGGACA

        130        140        150        160        170        180
AACCGTTTCA TTGGTTTGAG GAAGGTGACG GATCTGGGTA GAAACTGGAC TACTGCATCT

        190        200        210        220        230        240
GTTGGTAGTC TTGATGCCAT GGTGATGAGC CATTGCCATT GGAAAAGAGT GAATTCAGAT

        250        260        270        280        290        300
TCCAAGATTT GGTCAATGAT TGATTTTGTA AGATTGAGAT CGTAATCCTG ATACTCTTTG

        310        320        330        340        350        360
AGCCATGTTT CCAACAGTTC TTCGGAATCT GCCGGTGTGG AAACGAGTAT TTCGGAGTAC

        370        380        390        400        410        420
AATCTCGGTG GTTGCGTTAT CTGAGAGGAT GGTGTAGTGG TTTGATGTTG CTGTGTGAAA

        430        440        450        460        470        480
GATGATGCAG AGCTGATCAA CGATTCGAAC TGGGAGATCA CTTCGTTCAC TTCTTCCTGG

        490        500        510        520        530        540
TTCCCGTTAC CTGTTTGCGT TTCCTCATAC ATTGGTACGC TATCCTCATC TTCAGATAAC

        550        560        570        580        590        600
GAAATATCAA ACTCATCGGA ATCGGACGCG TCGTTCAAAT CGCCCTCATC CTTGGTAATG

        610        620        630        640        650        660
TTCTTGAACC GGTCGAGAAG GTTGAGAATC TCTGTCGGAA CACCACCCTG CGGCGTATAC

        670        680        690        700        710        720
CAGAACCAGA ATAAATTGTA GCACATCTTA ACTTTCTCTA AGGAAACATC TGAACTCTGA

        730        740        750        760        770        780
TCAACGCATT CCGTAAGTAT ACTGTTTGCC TTGTCTCTGG TGAATTTATG AGGGTAAGAC

        790        800        810        820        830        840
TCTGAGATCA TAAGTAACTG TTGAGCATCG AAGTTGTTGT AGTTTGAAAT TAGGGATCTG

        850        860        870        880        890        900
GAAAGATGCG GTACCACTGC TTTGATGACA TTATCTGGCG GGTTCAACGG TACCAATTCC

        910        920        930        940        950        960
TGCAAGAATA GCGAATCCAA CGGTTTTAAC TCAGAGTAAT GGTTGATCAA CTCGATGAAA

        970        980        990       1000       1010       1020
ACGTCCCAAT GGATGGATTG CATCAAGTGT TGATGTTCCA CCAAATTAAG ACAATATTTC

       1030       1040       1050       1060       1070       1080
GTAACGTTTT CGAGTGAAAC TGACACGGGC CTGCCCTCAG CACTCGTAGA CACGAGTAAC

       1090       1100       1110       1120       1130       1140
GTCTTGAGAC CTCTCGTACA GGGAAGCGAC ATATCGTTCA ATAGACTATG GAACAAAGTG

       1150       1160       1170       1180       1190       1200
TACACCGCAG CGATATCCTT GCATTTGCAA AACGATTGAA TAAGTGACGT CGATGCTAAA

       1210       1220       1230       1240       1250       1260
TCCTGGATAA GTACGCTGGT ATCGTGTAAG CCCATGAGAA CGACACGTTC CTCATCACTA
```

FIGURE 1

```
          1270        1280        1290        1300        1310        1320
GAAGCCGAAC  TGTTGTCTTC  AGTGGGGATT  GGTTCGACAT  TTTGCCAATT  GCTGTCGATG

          1330        1340        1350        1360        1370        1380
TACCCTTTCA  AAGCCATGTA  CCTTAAATCT  TCATCCTTGG  CAAGTAGATT  CATCGGGTGT

          1390        1400        1410        1420        1430        1440
GTTTGAAGTA  AGAATATTTG  CTTGTTTTTA  TGGTATCAAA  GGTATATGTT  GTAGAAGACA

          1450        1460        1470        1480        1490        1500
ATTTCCGGTA  ATCCAATTGT  CTGTCTGCTC  AGTTTAGCAC  ATGTATAGTA  CGTTGCACAT

          1510        1520        1530        1540        1550        1560
AGTCTACAAT  ATTCAGCATT  CAGCATTCAG  TATACAGCAT  ATGGCTAAAT  GATCACAAAT

          1570        1580        1590        1600        1610        1620
GTGATTGATG  ATTTGACACG  ACTAGAAAAG  AGAACGAAAA  AGGGAAATTC  ATGTCACGTG

          1630        1640        1650        1660        1670        1680
CGTTGGCACG  TGACATGGAA  TATCGAAGAA  AGAAAAAAAA  AAACGATCTC  GTCCTAGTGG

          1690        1700        1710        1720        1730        1740
AAGCCCAGAG  TCTGGTCCCC  CCGGAGTCTT  CCCAAAACAA  GAAGCTGACA  CATGTTGACA

          1750        1760        1770        1780        1790        1800
CAGAACACCC  CACAGCAAAT  GCACCACGCT  ACGTAGATCA  GGAAGCTTAA  CTCTAGCGAC

          1810        1820        1830        1840        1850        1860
CTGTCGCTCG  CCCCACAGAA  CCTCACCCGA  GAACCACACA  TTACACGCCG  CCAGCTCCCA

          1870        1880        1890        1900        1910        1920
CTATACTCAT  CTTGCTTCCC  TTAAGCGTTC  TCACGATTCG  TTCGCTGCCC  TTCTTCAAGA

          1930        1940        1950        1960        1970        1980
GTCTTCTGAT  TCTAATTCTC  ATTCGAAATC  CTCTACAGTT  AATGAATTGC  TTGACATGAC

          1990        2000        2010        2020        2030        2040
ATTCATTGTC  TCATGGTTTT  GGCTTTTTGG  CTTTTGTCTT  TTAAAGCTAT  ATCAACTTTA

          2050        2060        2070        2080        2090        2100
CATATAAATA TACGTCAAAA  GGGGATTCAT  TAATTAGAAA  ATTCTCTTTT  TCAATAGTTG

          2110        2120        2130        2140        2150        2160
CTATTCATTA  TCAATCTATT  CAACTCAATT  GGTTATTATT  TTCATCTTTT  TGTCATCCTA

          2170        2180        2190        2200        2210        2220
AACCATCAAC  AATATTTAAA TATATCTGTT  GCTACATTAA  GAGTTACTTC AGAAATAACA

          2230        2240        2250
AAAAAATCGA  TCAAGAATTA  ATAAAAATG
                                Met
```

FIGURE 1 (suite)

9

FIGURE 2

FIGURE 3

FIGURE 4

SalI

**A**

5'GGGAATTCGTCGA
CTTAACTCTAGCGACCTGTC 3'
3'----------TTCGAATTGAGATCGCTGGACAGGCAGCGGGGT-------------5'
5'------------------------------------------------------------3'
-467_____
HindIII

                                                                    1
3' -----------------------------------------------------------5'
5' -----------CAAAAAAATCGATCAAGAATTAATAAAAATG------3'
    3'  GTTTTTTTAGCTAGTTCTTAATT
                                        TCGAACTTAAGGG 5'
                                        _____    **B**
                                        HindIII

FIGURE 5

Oligodésoxynucléotide A
5'CAT<u>GTCGAC</u>TTTTTATTAATTCTTGATCGAT3'
      SalI
Oligodésoxynucléotide B
5'ATG<u>AAGCTT</u>AAATCTTCATCCTTGGC3'
     HindIII
Oligodésoxynucléotide C
5'GGGTGAGGTTCTGTGGGGCGAGCGACAGGTCGCTAGAGTTAAGC<u>A</u>TCCTGATC3'
(Position: 439 à 492)

Oligodésoxynucléotide D
5'GATCAGGA<u>T</u>GCTTAACTCTAGCGACCTGTCGCTCGCCCCACAGAACCTCACCC3'
(Position: 439 à 492)


```
HindIII    10        20        30        40        50        60
aagcttTTAA ATCTTCATCC TTGGCAAGTA GATTCATCGG GTGTGTTTGA AGTAAGAATA   60
ttcgaaAATT TAGAAGTAGG AACCGTTCAT CTAAGTAGCC CACACAAACT TCATTCTTAT

TTTGCTTGTT TTTATGGTAT CAAAGGTATA TGTTGTAGAA GACAATTTCC GGTAATCCAA  120
AAACGAACAA AAATACCATA GTTTCCATAT ACAACATCTT CTGTTAAAGG CCATTAGGTT

TTGTCTGTCT GCTCAGTTTA GCACATGTAT AGTACGTTGC ACATAGTCTA CAATATTCAG  180
AACAGACAGA CGAGTCAAAT CGTGTACATA TCATGCAACG TGTATCAGAT GTTATAAGTC

CATTCAGCAT TCAGTATACA GCATATGGCT AAATGATCAC AAATGTGATT GATGATTTGA  240
GTAAGTCGTA AGTCATATGT CGTATACCGA TTTACTAGTG TTTACACTAA CTACTAAACA

CACGACTAGA AAAGAGAACG AAAAAGGGAA ATTCATGTCA CGTGCGTTGG CACGTGACAT  300
GTGCTGATCT TTTCTCTTGC TTTTTCCCTT TAAGTACAGT GCACGCAACC GTGCACTGTA

GGAATATCGA AGAAAGAAAA AAAAAAACGA TCTCGTCCTA GTGGAAGCCC AGAGTCTGGT  360
CCTTATAGCT TCTTTCTTTT TTTTTTTGCT AGAGCAGGAT CACCTTCGGG TCTCAGACCA

CCCCCCGGAG TCTTCCCAAA ACAAGAAGCT GACACATGTT GACACAGAAC ACCCCACAGC  420
GGGGGGCCTC AGAAGGGTTT TGTTCTTCGA CTGTGTACAA CTGTGTCTTG TGGGGTGTCG

AAATGCACCA CGCTACGTAG ATCAGG<u>ATGC </u>TTAACTCTAG CGACCTGTCG CTCGCCCCAC  480
TTTACGTGGT GCGATGCATC TAGTCCT<u>A</u>CG AATTGAGATC GCTGGACAGC GAGCGGGGTG

AGAACCTCAC CCGAGAACCA CACATTACAC GCCGCCAGCT CCCACTATAC TCATCTTGCT  540
TCTTGGAGTG GGCTCTTGGT GTGTAATGTG CGGCGGTCGA GGGTGATATG AGTAGAACGA

TCCCTTAAGC GTTCTCACGA TTCGTTCGCT GCCCTTCTTC AAGAGTCTTC TGATTCTAAT  600
AGGGAATTCG CAAGAGTGCT AAGCAAGCGA CGGGAAGAAG TTCTCAGAAG ACTAAGATTA

TCTCATTCGA AATCCTCTAC AGTTAATGAA TTGCTTGACA TGACATTCAT TGTCTCATGG  660
AGAGTAAGCT TTAGGAGATG TCAATTACTT AACGAACTGT ACTGTAAGTA ACAGAGTACC

TTTTGGCTTT TTGGCTTTTG TCTTTTAAAG CTATATCAAC TTTACATATA AATATACGTC  720
AAAACCGAAA AACCGAAAAC AGAAAATTTC GATATAGTTG AAATGTATAT TTATATGCAG

AAAAGGGGAT TCATTAATTA GAAAATTCTC TTTTTCAATA GTTGCTATTC ATTATCAATC  780
TTTTCCCCTA AGTAATTAAT CTTTTAAGAG AAAAAGTTAT CAACGATAAG TAATAGTTAG

TATTCAACTC AATTGGTTAT TATTTTCATC TTTTTGTCAT CCTAAACCAT CAACAATATT  840
ATAAGTTGAG TTAACCAATA ATAAAGTAG  AAAAACAGTA GGATTTGGTA GTTGTTATAA

TAAATATATC TGTTGCTACA TTAAGAGTTA CTTCAGAAAT AACAAAAAAA TCGATCAAGA  900
ATTTATATAG ACAACGATGT AATTCTCAAT GAAGTCTTTA TTGTTTTTTT AGCTAGTTCT

ATTAATAAAA A<u>gtcgac</u>                                                 917
TAATTATTTT Tcagctg
           10 SalI     20        30        40        50        60
```


FIGURE 6

Figure 7

Figure 8

Figure 9

Figure 10

Figure 11

Figure 12

FIGURE 13

FIGURE 14

EP 0 511 912 A1

**Office européen des brevets** **RAPPORT DE RECHERCHE EUROPEENNE** Numero de la demande

EP 92 40 1206

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 361 991 (RHONE-POULENC SANTE) 4 Avril 1990<br>* revendications 13-16 * | 1-12 | C12N15/81<br>C12N1/19<br>C12P21/02 |
| Y | NUCLEIC ACIDS RESEARCH.<br>vol. 18, no. 2, 25 Janvier 1990, ARLINGTON, VIRGINIA US<br>page 365;<br>A. FOURNIER, R. FLEER, P. YEH AND J.-F. MAYAUX: 'The primary structure of the 3-phosphoglycerate kinase (PGK) gene from Kluyveromyces lactis'<br>* le document en entier * | 1-12 | |
| Y | BIOTECHNOLOGY<br>vol. 8, no. 2, Février 1990, NEW YORK US<br>pages 135 - 139;<br>J.A. VAN DEN BERG ET AL.: 'Kluyveromyces as a host for heterologous gene expression: expression and secretion of prochymosin'<br>* page 135, colonne 2, ligne 21 - page 135, colonne 2, ligne 39; figure 1 * | 1-12 | DOMAINES TECHNIQUES RECHERCHES (Int. Cl.5) |
| A | JOURNAL OF BASIC MICROBIOLOGY<br>vol. 28, no. 4, 1988, BERLIN, GERMANY<br>pages 211 - 220;<br>X.J. CHEN ET AL.: 'A gene-cloning system for Kluyveromyces lactis and isolation of a chromosomal gene required for killer toxin production'<br>* page 214, ligne 13 - page 214, ligne 32 * | | C12N<br>C12P |

Le présent rapport a été établi pour toutes les revendications

| Lieu de la recherche | Date d'achèvement de la recherche | Examinateur |
|---|---|---|
| LA HAYE | 22 JUIN 1992 | VAN PUTTEN A.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

22